# EUROPEAN PATENT SPECIFICATION

(11) **EP 2 914 093 B1**
(45) Date of publication and mention of the grant of the patent: **13.05.2020**
(21) Application number: 13792808.1
(22) Date of filing: 04.11.2013
(51) Int. Cl.: A01H 1/04, A01H 4/00, C12Q 1/68

(54) **EMBRYO SAMPLING FOR MOLECULAR ANALYSIS**
EMBRYO-PROBENNAHME FÜR MOLEKULARE ANALYSE
ECHANTILLONNAGE D'EMBRYON POUR L'ANALYSE MOLÉCULAIRE

(30) Priority: 05.11.2012 US 201261722399 P; 15.03.2013 US 201361786968 P
(43) Date of publication of application: 09.09.2015
(73) Proprietor: Pioneer Hi-Bred International, Inc., Johnston, Iowa 50131-1014 (US)
(72) Inventor: HUNTER, Clifford, Mililani Hawaii 96789 (US)
(74) Representative: Dehns
(86) International application number: PCT/US2013/068191
(87) International publication number: WO 2014/071271

(56) References cited:
- WO-A1-2012/143696
- WO-A2-2007/103786
- F. DOMINGUEZ ET AL: "The scutellum of germinated wheat grains undergoes programmed cell death: identification of an acidic nuclease involved in nucleus dismantling", JOURNAL OF EXPERIMENTAL BOTANY, vol. 63, no. 15, 1 September 2012 (2012-09-01), pages 5475-5485, XP055096769, ISSN: 0022-0957, DOI: 10.1093/jxb/ers199
- ISHIDA YUJI ET AL: "Agrobacterium-mediated transformation of maize", NATURE PROTOCOLS, NATURE PUBLISHING GROUP, GB, vol. 2, no. 7, 1 July 2007 (2007-07-01), pages 1614-1621, XP009148413, ISSN: 1750-2799, DOI: 10.1038/NPROT.2007.241 [retrieved on 2007-06-21]
- Biotechniques: "Rapid Identification of Transformed Wheat Using a Half-Seed PCR Assay", , 1 March 2002 (2002-03-01), XP055096691, Retrieved from the Internet: URL:http://www.biotechniques.com/multimedi a/archive/00010/02323st09_10762a.pdf [retrieved on 2014-01-15]
- B. H. LE ET AL: "Using Genomics to Study Legume Seed Development", PLANT PHYSIOLOGY, vol. 144, no. 2, 27 April 2007 (2007-04-27), pages 562-574, XP055096820, ISSN: 0032-0889, DOI: 10.1104/pp.107.100362
- J. W. Chandler: "Cotyledon organogenesis", JOURNAL OF EXPERIMENTAL BOTANY, vol. 59, no. 11, 13 June 2008 (2008-06-13) , pages 2917-2931, XP055445036, GB ISSN: 0022-0957, DOI: 10.1093/jxb/ern167

## Description

### FIELD OF THE INVENTION

The present disclosure relates to methods for sampling isolated embryos to identify embryos that can develop into plants that have desirable characteristics.

### BACKGROUND OF THE INVENTION

In traditional plant breeding, generations of plants based on known crosses or self-pollinations are planted and then tested to see if the lines or varieties are moving towards characteristics that are desirable in the marketplace. As can be appreciated and as is well known in the art, these experiments can be massive in scale. They involve a huge labor force ranging from scientists to field staff to design, plant, maintain, and conduct the experiments, which can involve thousands or tens of thousands of individual plants. They also require substantial land resources. Plots or greenhouses can take up thousands of acres of land. Not only does this tie up large amounts of land for months while the plants germinate, grow, and produce seed, during which time they may be sampled for laboratory or field testing, but then the massive amounts of seed must be individually tagged, harvested and processed.

A further complication is that much of the experimentation goes for naught. It has been reported in the literature that some seed companies discard 80-90% of the plants in any generation early on in the experiment. Thus, much of the land, labor and material resources expended for growing, harvesting, and post-harvest processing ultimately are wasted for a large percentage of the seed.

Timing pressures are also a factor. Significant advances in plant breeding have put more pressure on breeding programs to more quickly advance lines or varieties of plants for more and better traits and characteristics. The plant breeders and associated workers are thus under increasing pressure to more efficiently and effectively process these generations and to make more and earlier selections of plants which should be continued into the next generation of breeding.

A number of currently used practices have accelerated the gains produced by plant breeding with considerable cost savings. For instance, protocols for creating doubled haploids have eliminated the need for years of selfing and subsequent evaluations to achieve homozygous plants. Moreover, the power of genetic marker data, and the ease of obtaining such, has allowed plants that do not contain desired characteristics to be removed from the population prior to the expenditure of significant resources. Furthermore, recent practices have utilized laboratory based testing at the seed stage, often referred to as "kernel chipping" or "seed chipping", wherein seed is non-destructively tested to derive genetic, biochemical or phenotypic information (Sangtong, V. et al. (2001) Plant Molecular Biology Reporter 19:151-158). Testing seed prevents the need to grow the seed into immature plants, thereby saving time, space, and effort.

WO 2007/103786 discloses methods to facilitate germplasm improvement activities through the use of high throughput, non-destructive sampling of seeds.

WO 2012/143696 discloses a method of selecting propagatable plant material of atypical ploidy by sampling the endosperm of individual seeds or seedlings without damaging the embryo and selecting the individuals in which the ploidy of the endosperm is abnormal.

However, there are still improvements that can be made. In particular, there is a need for methods to molecularly characterize embryos even earlier in development, particularly as part of the doubled haploid process or other processes that utilize embryo rescue techniques.

### SUMMARY OF THE INVENTION

The present disclosure relates to methods for facilitating plant improvement activities through the use of embryo sampling. Through the process described herein, it is possible to test individual isolated embryos and select only those embryos that can develop into plants that possess one or more desired characteristics. This allows for new and more efficient methods for plant improvement and management, which lead to improved breeding populations.

Methods of analyzing an isolated embryo of a monocot plant are provided herein. In such methods, a piece of scutellum tissue is excised from an isolated immature embryo, wherein said excision does not cause a significant reduction in the germination potential of the embryo. Nucleic acids extracted from the piece of scutellum tissue are then analyzed, optionally after amplification, for the presence or absence of one or more characteristics indicative of at least one genetic trait. The isolated embryo is viable and able to germinate into a plant after said piece of scutellum tissue is excised.

The isolated embryo may be from maize, sorghum, wheat, rice, barley, oats, rye, millet, sugar cane, triticale, or switchgrass. The isolated embryo may be obtained directly from a seed, or it may be derived from other tissues. The isolated embryo may be fresh or cooled. The isolated embryo may be of any ploidy such as but not limited to a haploid, diploid, doubled haploid, aneuploidy, tetraploid, hexaploid, or octaploid.

The piece of scutellum tissue excised from the isolated embryo may be lyophilized, fresh, frozen, or cooled after sampling.

The piece of scutellum tissue may be equal to or larger than a single nucleus.

Excision of the scutellum tissue may occur by any means known in the art such as but not limited to: a drill bit, a water jet, a laser, a single blade, a set of opposing blades, a syringe, a core sampler (coring tool), a scalpel, a small diameter wire, a small diameter textured wire rope, a spatula, and a swab. Excision may be performed manually or by an automated process.

The isolated embryo may be positioned meristem down wherein the piece is excised from the opposite end of the meristem. Positioning may be performed manually or in an automated fashion using mechanical movement, actuation, etc. Automation may include using robotics, vision systems, or a combination of both.

The piece of scutellum tissue may be analyzed for one or more characteristics selected from the group consisting of: a genetic marker, a single nucleotide polymorphism, a simple sequence repeat, a restriction fragment length polymorphism, a haplotype, a tag SNP, an allele of a genetic marker, a gene, a DNA-derived sequence, an RNA-derived sequence, a promoter, a 5' untranslated region of a gene, a 3' untranslated region of a gene, microRNA, siRNA, a QTL, a satellite marker, a transgene, mRNA, ds mRNA, a transcriptional profile, a methylation pattern, and ploidy level.

Methods of analyzing an isolated embryo of a dicot plant are also disclosed. In such methods, a piece of cotyledon tissue is excised from an isolated embryo, wherein said excision does not cause a significant reduction in the germination potential of the embryo. The piece of cotyledon tissue is then analyzed for the presence or absence of one or more characteristics indicative of at least one genetic trait.

The isolated embryo may be immature, and it may be canola, soybean, sunflower, alfalfa, or cotton. The isolated embryo may be obtained directly from a seed, or it may be derived from other tissues. The isolated embryo may be fresh or cooled. The isolated embryo is viable and is able to germinate into a plant after said piece of cotyledon tissue is excised. The isolated embryo may be of any ploidy such as but not limited to a haploid, diploid, doubled haploid, aneuploidy, tetraploid, hexaploid, or octaploid.

The piece of cotyledon tissue excised from the isolated embryo may be lyophilized, fresh, frozen, or cooled after sampling.

The piece of cotyledon tissue may be equal to or larger than a single nucleus.

Excision of the cotyledon tissue may occur by any means known in the art such as but not limited to: a drill bit, a water jet, a laser, a single blade, a set of opposing blades, a syringe, a core sampler (coring tool), a scalpel, a small diameter wire, a small diameter textured wire rope, a spatula, and a swab. Excision may be performed manually or by an automated process.

The piece of cotyledon tissue may be analyzed for one or more characteristics selected from the group consisting of: a genetic marker, a single nucleotide polymorphism, a simple sequence repeat, a restriction fragment length polymorphism, a haplotype, a tag SNP, an allele of a genetic marker, a gene, a DNA-derived sequence, an RNA-derived sequence, a promoter, a 5' untranslated region of a gene, a 3' untranslated region of a gene, microRNA, siRNA, a QTL, a satellite marker, a transgene, mRNA, ds mRNA, a transcriptional profile, a methylation pattern, and ploidy level.

### DETAILED DESCRIPTION

As used herein and in the appended claims, the singular forms "a", "an", and "the" include plural reference unless the context clearly dictates otherwise. Thus, for example, reference to "a plant" includes a plurality of such plants, reference to "a cell" includes one or more cells and equivalents thereof known to those skilled in the art, and so forth.

### As used herein:

"Callus" refers to a dedifferentiated proliferating mass of cells or tissue.

The phrases "contacting", "comes in contact with" or "placed in contact with" can be used to mean "direct contact" or "indirect contact". For example, the medium comprising a doubling agent may have direct contact with the haploid cell or the medium comprising the doubling agent may be separated from the haploid cell by filter paper, plant tissues, or other cells thus the doubling agent is transferred through the filter paper or cells to the haploid cell.

A "diploid" plant has two sets (genomes) of chromosomes and the chromosome number (2n) is equal to that in the zygote.

A "doubled haploid" or doubled haploid plant or cell is one that is developed by the doubling of a haploid set of chromosomes. A plant or seed that is obtained from a doubled haploid plant that is selfed any number of generations may still be identified as a doubled haploid plant. A doubled haploid plant is considered a homozygous plant. A plant is considered to be doubled haploid if it is fertile, even if the entire vegetative part of the plant does not consist of the cells with the doubled set of chromosomes. For example, a plant will be considered a doubled haploid plant if it contains viable gametes, even if it is chimeric.

A "doubled haploid embryo" is an embryo that has one or more cells that contain 2 sets of homozygous chromosomes.

"Embryogenesis" may be defined as the process of embryo initiation, proliferation and/or development.

"Embryogenic," in the context of cells or tissues, means that the cells or tissues can be induced to form viable plant embryos under appropriate culture conditions.

"Germination" refers to the process of development of the embryo of a seed into a plant. Germination may occur in vitro or in vivo.

"Germination potential" can be defined as the capacity of an embryo to complete germination. The phrase "without a significant reduction in germination potential" refers to the fact that a normal plant will develop. A normal plant can be defined as one that can successfully set seed.

A "haploid" plant has a single set (genome) of chromosomes and the chromosome number (n) is equal to that in the gamete.

An "isolated embryo" is an embryo that is not associated with a seed. This may occur due to removal of an embryo from a seed or because the embryo is derived from other tissues through somatic or gametic (microspore) embryogenesis.

A "mature" embryo refers to an embryo that has completed embryogenesis wherein said mature embryo is dehydrated and metabolically dormant. An "immature" embryo refers to an embryo from the initiation of egg cell division through the end of embryogenesis.

The term "mature somatic embryo" refers to a fully-developed embryo derived from somatic tissue, with evidence of root and shoot apices and exhibiting a bipolar structure. In monocots, the mature somatic embryo will have a scutellum.

The term "medium" includes compounds in liquid, gas, or solid state.

The terms "monocot" and "monocotyledonous plant" are used interchangeably herein.

The terms "dicot" and "dicotyledonous plant" are used interchangeably herein.

As used herein, the term "plant" includes reference to whole plants, plant organs (e.g., leaves, stems, roots, etc.), seeds and plant cells and progeny of same. "Plant cell", as used herein includes, without limitation, seeds, suspension cultures, embryos, meristematic regions, callus tissue, leaves, roots, shoots, gametophytes, sporophytes, pollen, and microspores.

The term "primary somatic embryo" refers to a somatic embryo that originates from tissues other than those of another somatic embryo. By "somatic embryo" is meant an embryo formed *in vitro* from somatic cells or embryogenic cells by mitotic cell division.

"Scutellum sampling" refers to the excision of a scutellar portion of an embryo.

"Cotyledon sampling" refers to the excision of a cotyledon portion of an embryo.

The term "somatic embryogenesis" refers to the process of initiation and development of embryos *in vitro* from plant cells and tissues absent sexual reproduction.

*Turning now to the* disclosure:
Embryo sampling permits molecular characterization early in plant development, allowing selections of a desired genotype to be made weeks, even months earlier than other, currently employed, sampling methods. Consequently, resources can be focused earlier on embryos that have the highest probability of developing into desirable plants.

The scutellum is the modified cotyledon of plants in the Poaceae family, a large family of monocotyledous flowering plants. It is a shield-shaped structure surrounding the embryonic axis and it has diverse functions. During seed formation, the scutellum acts as a storage organ accumulating mainly lipids but also proteins and starch. Then, during germination, the scutellum secretes both hormones that induce the production of hydrolytic enzymes in the aleurone layer and enzymes that assist in the digestion of endosperm reserves. In addition, the scutellum also transports the digested nutrients from the endosperm to the embryo axis.

The scutellum has a high cell density and therefore, more nuclei per unit of tissue as compared to leaf tissue, resulting in a higher DNA concentration. The high density of DNA makes scutellum tissue an excellent source for genomic DNA extraction; however, previous efforts have not focused on the scutellum tissue as a source of sampling because it was thought that removing a portion of the scutellum would have severe implications on development of the embryo.

Methods of analyzing an embryo of a monocot plant are provided, in which a piece of scutellum tissue is removed or excised from the isolated immature embryo. Cotyledons of isolated embryos of dicot plants may also be sampled in a similar fashion. As such, methods of analyzing an embryo of a dicot plant are also disclosed herein, in which a piece of cotyledon tissue is removed or excised from the isolated embryo.

When excising a piece of scutellum or cotyledon tissue from an isolated embryo, care must be taken to remove the tissue without damaging the meristematic regions of the embryo. This will allow the embryo to continue to develop, with minimal disruption, into a normal plant (i.e. the embryo does not have a significant reduction in germination potential).

The methods of the current disclosure may further comprise treating the sampled embryos to maintain germination potential. Such treatment may generally include any means known in the art for protecting an embryo, or a plant or plantlet derived from an embryo, from environmental conditions while in storage or transport. For example, the sampled embryos may be treated with a polymer and/or a fungicide to protect the sampled embryo while in storage or in transport.

The methods of the current disclosure may further comprise attaching an identifier to the receptacle containing the sample as well as to the receptacle containing the isolated embryo from which the sample was excised. This maintains the identity of the sample and embryo, allowing them to be properly tracked such that the sample is always associated with the embryo from which it was excised. The identifier may be a printed barcode or label.

In the methods of the disclosure, a sample may be excised or removed from an isolated immature embryo of a monocot plant wherein said sample is a piece of scutellum tissue. The monocot plant may be but is not limited to maize, sorghum, wheat, rice, barley, oats, rye, millet, sugar cane, triticale, or switchgrass.

The isolated embryo may be obtained from a seed (i.e. zygotic embryogenesis) or may be "derived from other tissues" through somatic or gametic (microspore) embryogenesis. Somatic embryogenesis relates to embryogenesis arising from somatic cells (i.e. vegetative or non-gametic cells), namely from isolated somatic explants whereas gametic embryogenesis relates to embryogenesis arising from gametic cells (i.e. microspores). Since somatic and gametic cells are not naturally embryogenic, such cells must be induced to become embryogenic. Conversion to embryogenic cells may be achieved by external stimuli such as auxin, cytokinin, pH shifts, growth regulators, and heavy metal ions (Yeung, 1995 In: Thorpe TA (ed) In Vitro Embryogenesis in Plants (pp. 205-249; Dodeman et al. (1997) J. Exp. Bot. 48:1493-1509.

The isolated embryo must be viable and able to germinate into a plant after the piece of scutellum or cotyledon tissue is excised, for monocot and dicot embryos, respectively. The isolated embryo may be of any ploidy that can exist in a monocot plant of the disclosure. For example, the isolated embryo may be a haploid, diploid, doubled haploid, aneuploid, tetraploid, hexaploid, or octaploid.

It can be appreciated that an isolated embryo may or may not be sampled immediately upon isolation. Thus, the isolated embryo may be fresh or cooled prior to and/or during sampling.

Sampling methods may be referred to herein in different terms (used interchangeably herein), such as, for example, sampling, excising, chipping, clipping, slicing, cutting, snipping, or removing a sample. Sampling of the scutellum may occur by any means known in the art such as but not limited to: a drill bit, a water jet, a laser, a single blade, a set of opposing blades, a syringe, a core sampler (coring tool), a scalpel, a small diameter wire, a small diameter textured wire rope, a spatula, and a swab. Moreover, sampling may be performed manually or by an automated process.

Automation of excision of the piece of scutellum or cotyledon tissue may include one or more of the following: a) determining the location and orientation of an isolated embryo from a plurality of isolated embryos using an automated vision instrument; b) determining physical specifications of an isolated embryo (e.g. length, width, surface area, thickness, and shape) using an automated vision instrument; c) orienting an isolated embryo into/onto a prescribed location using pneumatic/vacuum gripping or mechanical/physical manipulation; d) orienting the excision tool relative to an isolated embryo either physically, or in the case of a laser-guided excision tool, optically; e) actuating the tool or moving an isolated embryo through the tool to remove the sample; f) placing the sample and remaining embryo into separate containers; g) tracking the sample and remaining embryo in relation to each other for use and selection in the breeding process; h) cleaning and/or sterilization of the tool to prevent cross-contamination or bacterial growth; i) disposal of tool if cleaning and/or sterilization is not performed; j) placement of a fragment of a tool into a container for direct analysis wherein the sample is in/on said fragment such that the fragment of the tool does not interfere with the extraction (e.g. as with a wire used to scrape or pierce an isolated embryo which is then cut into a lab plate for DNA extraction); and k) rinsing of the tool in extraction media.

If a small diameter wire is used, the small diameter wire may be less than 1 mm in diameter, and the wire may be cold or hot. The wire may also be shaped into a structure that facilitates sampling.

Any laser known to one or ordinary skill in the art for cutting biological tissue may be used. Lasers that cut in the Nanosecond and Picosecond range are suitable for use in the methods of the disclosure. Another type of laser that may be used is a Femtosecond laser. Femtosecond lasers have pulse widths in the femtosecond domain (1 fs = 10⁻¹⁵ seconds), which produces a narrow kerf and minimizes the heat affected zone, thereby avoiding heat damage to the material being cut.

The "sample" may also be described herein as a cell, a nuclei, a piece, a clip, a chip, a sliver, a part, a portion, a fragment, a section, a slice, or any other term referring to a monolithic piece that may be separated from an embryo without destroying the viability of the embryo or its potential for developing into a healthy plant (i.e. germination potential). The sample excised from the isolated embryo may be lyophilized, fresh, frozen, or cooled after sampling.

The size of the piece of scutellum tissue excised from the isolated embryo may be equal to or larger than a single nucleus. The piece of scutellum tissue may also be a sloughed cell or less than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% of the total scutellum. The size of the piece may be any portion of the scutellum that does not cause a significant reduction in germination potential of the isolated embryo.

The size of the piece of cotyledon tissue excised from the isolated dicot embryo may be equal to or larger than a single nucleus. The piece of cotyledon tissue may also be a sloughed cell or less than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% of the total amount of cotyledon tissue. The piece may be from one of the two cotyledons or from both of the cotyledons.

The sample may be taken from any area of the scutellum, and the isolated monocot embryo may be positioned meristem down wherein the piece of scutellum is excised from the opposite end of the meristem.

Similarly, the sampling may be taken from any area of the cotyledon(s) of the isolated dicot embryo. The isolated dicot embryo may be positioned in such a way to avoid damage to the meristem.

Positioning may be performed manually or in an automated fashion using mechanical movement, actuation, etc. Automation may include using robotics, vision systems, or a combination of both.

The piece of scutellum or cotyledon tissue may be analyzed for one or more characteristics indicative of a genetic trait. The genetic trait may include but is not limited to a genetic marker, a single nucleotide polymorphism, a simple sequence repeat, a restriction fragment length polymorphism, a haplotype, a tag SNP, an allele of a genetic marker, a gene, a DNA-derived sequence, an RNA-derived sequence, a promoter, a 5' untranslated region of a gene, a 3' untranslated region of a gene, microRNA, siRNA, a QTL, a satellite marker, a transgene, mRNA, ds mRNA, a transcriptional profile, a methylation pattern, and ploidy level.

DNA may be extracted from the sample using any DNA extraction method known to those of skill in the art which will provide sufficient DNA yield, DNA quality, PCR response, and sequencing methods response. This may include but is not limited to: Extract N Amp (Sigma-Aldrich), standard CTAB protocol, HotShot methods, etc. In addition, the extracted DNA may be amplified after extraction using any amplification method known to those skilled in the art.

Further, RNA may be extracted from the sample using any RNA extraction method known to those of skill in the art which will provide sufficient RNA yield, RNA quality, PCR response, and sequencing methods response. In addition, the extracted RNA may be amplified after extraction using any amplification method known to those skilled in the art.

The extracted nucleic acids may be analyzed for the presence or absence of a suitable genetic polymorphism. A wide variety of genetic markers for the analysis of genetic polymorphisms are available and known to those of skill in the art. As used herein, genetic markers include, but are not limited to, simple sequence repeats (SSRs), single nucleotide polymorphisms (SNPs), insertions or deletions (indels), transcriptional profiles, and nucleic acid sequences. A nucleic acid analysis for the presence or absence of the genetic marker may be used for the selection of embryos as part of a plant improvement or breeding program. The analysis may be used to select embryos that house genes, QTL, alleles, or haplotypes of interest. Analysis methods are known in the art and include, but are not limited to, PCR-based detection methods (such as for example, TaqMan assays), microarray methods, and nucleic acid sequencing methods. The genes, alleles, QTL, or haplotypes may also be identified using newer techniques of molecular biology.

### Methods using embryo sampling

The methods of the present disclosure use embryo sampling to contribute to germplasm improvement activities including but not limited to: economization of doubled haploid programs by selecting only preferred embryos for doubling, analysis of haploid and doubled haploid material for genotypic characteristics, trait integration and evaluation, and marker-assisted breeding.

Doubled haploid (DH) plants provide an invaluable tool to plant breeders, particularly for generating inbred lines. A great deal of time is spared as homozygous lines are essentially instantly generated, negating the need for multigenerational conventional breeding.

However, it is well known in the art that the DH production process is inefficient and can be quite labor-intensive. While doubled haploid plants can occur spontaneously in nature, this is extremely rare. Most research and breeding applications rely on artificial methods of DH production. The initial step involves the haploidization of the plant which results in the production of a population comprising haploid seed. Non-homozygous lines are crossed with an inducer parent, resulting in the production of haploid seed. Seed that has a haploid embryo, but normal triploid endosperm, advances to the second stage. That is, haploid seed and plants are any plant with a haploid embryo, independent of the ploidy level of the endosperm. After selecting haploid seeds from the population, the selected seeds undergo chromosome doubling to produce doubled haploid seeds. A spontaneous chromosome doubling in a cell lineage will lead to normal gamete production or the production of unreduced gametes from haploid cell lineages. Application of a chemical compound, such as colchicine, can be used to increase the rate of diploidization. Colchicine binds to tubulin and prevents its polymerization into microtubules, thus arresting mitosis at metaphase, and can be used to increase the rate of diploidization, i.e. doubling of the chromosome number. These chimeric plants are self-pollinated to produce diploid (doubled haploid) seed. This DH seed is cultivated and subsequently evaluated and used in hybrid testcross production.

The methods of the present disclosure facilitate the potential for selection at the haploid as well as the doubled haploid stage. As part of a doubled haploid program, immature haploid embryos are isolated, and the haploid embryos are then contacted with a doubling agent such as colchicine or other agent known in the art. Sampling may be performed prior to exposure to the doubling agent or afterwards. With the former, embryos sampled prior to doubling can be identified as candidates for doubling based on the outcome of sampling. With the latter, as soon as the doubling phase has been completed it is necessary to transfer the selected embryos from the doubling medium to a medium devoid of doubling agent, such as for example, germination medium. At this stage it is convenient and inexpensive to remove a piece of scutellum or cotyledon tissue for molecular analysis. The methods presented herein thereby allow for advancement decisions to be made early in the doubled haploid process.

The methods of the disclosure can be easily integrated into any embryo rescue process in which an embryo is isolated and then cultured.

For example, in plant breeding, the use of embryo rescue techniques significantly decreases the time it takes to transfer a beneficial trait from a donor parent to a recurrent parent with the desired genetic background (Wang et al. 2011. Plant Breeding. 130:569-573). The techniques involve culturing immature embryos on a nutrient media that may or may not be supplemented with a selective agent to screen for transgenic explants, transplanting the embryos or plantlets derived thereform to a controlled growth environment for a sufficient period of time, and then transplanting the plantlets to the field. Prior to the expenditure of resources, it is convenient and inexpensive to remove a piece of scutellum or cotyledon tissue from the immature embryos for molecular analysis in order to identify those embryos that will produce plants with desirable traits.

### EXAMPLES

The present invention is further illustrated in the following Examples, in which parts and percentages are by weight and degrees are Celsius, unless otherwise stated. It should be understood that these Examples, while indicating embodiments of the invention, are given by way of illustration only. From the above discussion and these Examples, one skilled in the art can ascertain the essential characteristics of this invention, and can make various changes and modifications of the invention to adapt it to various usages and conditions. Thus, various modifications of the invention in addition to those shown and described herein will be apparent to those skilled in the art from the foregoing description. Such modifications are also intended to fall within the scope of the appended claims.

### EXAMPLE 1

### Scutellum Sampling to Obtain DNA for Molecular Analysis (Monocots) Scutellum sampling

Scutellum tissue was excised from immature embryos of about 2-4 mm in size and then placed on media. The samples were placed in the refrigerator at least one week prior to extracting DNA, and the remaining portions of the maize embryos were planted in the shadehouse.

### DNA extraction and marker testing

Samples were removed from the media with tweezers and rinsed in HPLC water and then patted dry. Samples were then placed in sample tubes within a 96 well plate. Leaf tissue of unknown genotype was also added to unoccupied sample tubes in the 96 well plate as a means for comparison. Two replicate plates were made from the same source plate. An additional grinding step was utilized to ensure that the samples were ground well. Two different extraction protocols were used on each tissue type, the HotShot DNA extraction protocol and the Sbeadex extraction protocol. Thirty two SNP markers were initially tested using an Invader Plus platform.

The results obtained show that there is enough DNA extracted from each scutellum sample to perform molecular marker analysis, and in fact, the scutellum samples amplified with the same "intensity' as the leaf samples on the same plate.

### Performance of plants derived from sampled embryos

Development was delayed within the first week of germination; however, the delay was temporary and could not be detected a week after potting in the shadehouse.

### EXAMPLE 2

### Comparative Analysis Between Scutellum Sampling and Leaf Sampling for Molecular Analysis

A sample of scutellum tissue was excised from each immature embryo using a new, sterile scalpel. The samples were placed into field plates and then put in tubes using new, sterile cotton swabs. Leaf samples from doubled haploid plants were also collected in the field and then frozen. Two leaf punches were submitted per sample.

Two different extraction protocols were used on each tissue type, the HotShot DNA extraction protocol and the Sbeadex extraction protocol. Extracted DNA was run with forty-eight production SNP markers. Concordance was within production standards with only 0.22% flips (i.e. incorrect allele calls). Moreover, both leaf and scutellum samples performed within production standards of below 5% NF (not found). Heterozygotes (Hets) were scored as equivocal as the samples were doubled haploid samples.

**Table 1: Marker performance: comparison between scutellum and leaf samples**

| | **Extraction Protocol** | |
|---|---|---|
| | **Hot Shot** | **SbeadEx** |
| ***Scutellum*** | | |
| Low Signal | 1.51% | 0.27% |
| %Het | 0.10% | 0.05% |

| ***Leaf*** | | |
|---|---|---|
| Low Signal | 1.20% | 0.17% |
| %Het | 0.36% | 2.42% |

### EXAMPLE 3

### Cotyledon Sampling (Dicot Embryos) to Obtain DNA for Molecular Analysis

Cotyledon tissue was excised from immature microspore-derived canola embryos and placed on media. Samples were removed from the media with tweezers and rinsed in HPLC water and then patted dry. Samples were then placed in sample tubes within a 96 well plate. Leaf tissue of unknown genotype was also added to unoccupied sample tubes in the 96 well plate as a means for comparison. Two replicate plates were made from the same source plate. An additional grinding step was utilized to ensure that the samples were ground well. Two different extraction protocols were used on each tissue type, the HotShot DNA extraction protocol and the Sbeadex extraction protocol. Eight SNP markers were tested using an Invader Plus platform.

Extracted DNA (using either the Sbeadex or Hotshot extraction method) was run with eight production SNP markers. Results are shown in Table 3. The Sbeadex method resulted in a high %NF (i.e. not found). The Hotshot extraction method provided results slightly out of acceptable range (<5% NF) for the small immature embryos, but this was still better than the results obtained with leaf tissue. The "small" samples performed better than the "large" samples. Overall, the results showed that there is enough DNA extracted from each cotyledon sample to perform molecular marker analysis.

With respect to further development of the embryos, no unexpected developmental events were observed.

**Table 3: Results of SNP marker analysis using DNA obtained from cotyledon tissue**

| | **Hot Shot** |
|---|---|
| ***Small samples from immature embryo*** | |
| Low Signal | 4.17% |
| %Het | 0.97% |
| %NF | 5.14% |

| ***Large samples from immature embryo*** | |
|---|---|
| Low Signal | 7.89% |
| %Het | 0.22% |
| %NF | 8.11% |

| ***Leaf*** | |
|---|---|
| Low Signal | 6.03% |
| %Het | 0.60% |
| %NF | 6.63% |

## Claims

1. A method for analyzing an isolated embryo of a monocot plant comprising:
a. excising a piece of scutellum tissue from an isolated immature embryo wherein said excision does not cause a significant reduction in germination potential of said isolated embryo; and
b. analyzing nucleic acids extracted, and optionally amplified, from the piece of scutellum tissue for the presence or absence of one or more characteristics indicative of at least one genetic trait,
wherein said isolated embryo is able to germinate into a plant after said piece of tissue is excised.

2. The method of claim 1, wherein said monocot plant is maize, sorghum, wheat, rice, barley, oats, rye, millet, sugar cane, triticale, or switchgrass.

3. The method of claim 1 or claim 2, wherein said isolated embryo is obtained from a seed or is derived from other tissues.

4. The method of any one of claims 1 to 3, wherein said isolated embryo is fresh or cooled.

5. The method of any one of claims 1 to 4, wherein said piece of scutellum tissue is lyophilized, fresh, frozen, or cooled after sampling.

6. The method of any one of claims 1 to 5, wherein said piece of scutellum tissue is equal to or larger than a single nucleus.

7. The method of any one of claims 1 to 6, wherein said excision is performed using a tool selected from the group consisting of: a drill bit, a water jet, a laser, a single blade, a set of opposing blades, a syringe, a core sampler (coring tool), a scalpel, a small diameter wire, a small diameter textured wire rope, a spatula, and a swab.

8. The method of any one of claims 1 to 7, wherein said excision is performed by an automated process.

9. The method of any one of claims 1 to 8, wherein said piece of scutellum tissue is analyzed for one or more characteristics selected from the group consisting of: a genetic marker, a single nucleotide polymorphism, a simple sequence repeat, a restriction fragment length polymorphism, a haplotype, a tag SNP, an allele of a genetic marker, a gene, a DNA-derived sequence, an RNA-derived sequence, a promoter, a 5' untranslated region of a gene, a 3' untranslated region of a gene, microRNA, siRNA, a QTL, a satellite marker, a transgene, mRNA, ds mRNA, a transcriptional profile, a methylation pattern, and ploidy level.

## Patentansprüche

1. Verfahren zur Analyse eines isolierten Embryos einer einkeimblättrigen Pflanze, umfassend:
a. Herausschneiden eines Stücks Scutellum-Gewebe aus einem isolierten unreifen Embryo, wobei die Exzision keine signifikante Verringerung des Keimungspotentials des isolierten Embryos bewirkt; und
b. Analysieren von Nukleinsäuren, die aus dem Stück Scutellum-Gewebe extrahiert und gegebenenfalls amplifiziert wurden, auf das Vorhandensein oder Fehlen eines oder mehrerer Merkmale, die auf mindestens ein genetisches Merkmal hinweisen,
wobei der isolierte Embryo in der Lage ist, zu einer Pflanze zu keimen, nachdem das Gewebestück herausgeschnitten wurde.

2. Verfahren nach Anspruch 1, wobei die einkeimblättrige Pflanze Mais, Sorghum, Weizen, Reis, Gerste, Hafer, Roggen, Hirse, Zuckerrohr, Triticale oder Rutenhirse ist.

3. Verfahren nach Anspruch 1 oder Anspruch 2, wobei der isolierte Embryo aus einem Samen gewonnen wird oder aus anderen Geweben entnommen wird.

4. Verfahren nach einem der Ansprüche 1 bis 3, wobei der isolierte Embryo frisch oder gekühlt ist.

5. Verfahren nach einem der Ansprüche 1 bis 4, wobei das Stück Scutellum-Gewebe nach der Probenahme frisch, gefroren oder gekühlt lyophilisiert wird.

6. Verfahren nach einem der Ansprüche 1 bis 5, wobei das Stück Scutellum-Gewebe gleich oder größer als ein einzelner Nukleus ist.

7. Verfahren nach einem der Ansprüche 1 bis 6, wobei das Herausschneiden mit einem Werkzeug durchgeführt wird, das aus der Gruppe ausgewählt werden, die besteht aus: einem Bohrer, einem Wasserstrahl, einem Laser, einer einzelne Klingen, einem Satz gegenüberliegender Klingen, einer Spritze, einem Kernprobenehmer (Kernbohrwerkzeug), einem Skalpell, einem Draht mit kleinem Durchmesser, einem strukturiertes Drahtseil mit kleinem Durchmesser, einem Spatel und einem Tupfer.

8. Verfahren nach einem der Ansprüche 1 bis 7, wobei das Herausschneiden durch einen automatisierten Prozess durchgeführt wird.

9. Verfahren nach einem der Ansprüche 1 bis 8, wobei das Stück Scutellum-Gewebe auf ein oder mehrere Merkmale analysiert wird, die aus der Gruppe ausgewählt werden, die besteht aus: einem genetischen Marker, einem Einzelnukleotid-Polymorphismus, einer einfachen Sequenzwiederholung, einem Restriktionsfragmentlängen-Polymorphismus, einem Haplotyp, einem Tag SNP, einem Allel eines genetischen Markers, einem Gen, einer von der DNA abgeleiteten Sequenz, einer von der RNA abgeleiteten Sequenz, einem Promotor, einem 5' untranslatierten Bereich eines Gens, einem 3' untranslatierten Bereich eines Gens, mikroRNA, siRNA, einem QTL, einem Satellitenmarker, einem Transgen, mRNA, ds mRNA, einem Transkriptionsprofil, einem Methylierungsmuster und einem Ploidiegrad.

## Revendications

1. Procédé pour analyser un embryon isolé d'une plante monocotylédone comprenant les étapes consistant à :
a. exciser un morceau de tissu de scutellum d'un embryon immature isolé dans lequel ladite excision n'entraîne pas une réduction considérable de potentiel de germination dudit embryon isolé ; et
b. analyser des acides nucléiques extraits, et facultativement amplifiés, du morceau de tissu de scutellum pour la présence ou l'absence d'une ou plusieurs caractéristiques indicatives d'au moins un trait génétique,
dans lequel ledit embryon isolé est capable de germer en une plante après que ledit morceau de tissu a été excisé.

2. Procédé selon la revendication 1, dans lequel ladite plante monocotylédone est du maïs, du sorgho, du blé, du riz, de l'orge, de l'avoine, du seigle, du millet, de la canne à sucre, du triticale ou du panic érigé.

3. Procédé selon la revendication 1 ou la revendication 2, dans lequel ledit embryon isolé est obtenu d'une graine ou est dérivé d'autres tissus.

4. Procédé selon l'une quelconque des revendications 1 à 3, dans lequel ledit embryon isolé est frais ou refroidi.

5. Procédé selon l'une quelconque des revendications 1 à 4, dans lequel ledit morceau de tissu de scutellum est lyophilisé, frais, gelé ou refroidi après l'échantillonnage.

6. Procédé selon l'une quelconque des revendications 1 à 5, dans lequel ledit morceau de tissu de scutellum est de taille égale ou supérieure à un noyau unique.

7. Procédé selon l'une quelconque des revendications 1 à 6, dans lequel ladite excision est effectuée en utilisant un outil sélectionné à partir du groupe constitué par : une mèche de forage, un jet d'eau, un laser, une lame unique, un ensemble de lames opposées, un seringue, une sonde de carottage (un outil de carottage), un scalpel, un câble de petit diamètre, un câble métallique texturé de petit diamètre, une spatule, et un tampon.

8. Procédé selon l'une quelconque des revendications 1 à 7, dans lequel ladite excision est effectuée par un processus automatisé.

9. Procédé selon l'une quelconque des revendications 1 à 8, dans lequel ledit morceau de tissu de scutellum est analysé pour une ou plusieurs caractéristiques sélectionnées à partir du groupe constitué par : un marqueur génétique, un polymorphisme de nucléotide simple, une répétition de séquence simple, un polymorphisme de longueur de fragment de restriction, un haplotype, une étiquette SNP, un allèle d'un marqueur génétique, un gène, une séquence dérivée d'ADN, une séquence dérivée d'ARN, un promoteur, une région 5' non traduite d'un gène, une région 3' non traduite d'un gène, un micro-ARN, un petit ARN interfèrent, un QTL (locus de caractères quantitatifs), un marqueur satellite, un transgène, un ARNm, un ARNm à double brin, un profil transcriptionnel, un profil de méthylation, et un degré de ploïdie.
